# EUROPEAN PATENT APPLICATION

(11) **EP 4 014 872 A1**
(43) Date of publication of application: **22.06.2022**
(21) Application number: 20214175.0
(22) Date of filing: 15.12.2020
(51) Int. Cl.: A61B 6/00

(54) **PORTABLE X-RAY DETECTOR**

(71) Applicant: Koninklijke Philips N.V., 5656 AG Eindhoven (NL)
(72) Inventor: VON BERG, Jens, 5656 AE Eindhoven (NL); BUELOW, Thomas, 5656 AE Eindhoven (NL); YOUNG, Stewart Matthew, 5656 AE Eindhoven (NL); GOOßEN, André, 5656 AE Eindhoven (NL); WIEBERNEIT, Nataly, 5656 AE Eindhoven (NL); HARDER, Tim, 5656 AE Eindhoven (NL)
(74) Representative: Philips Intellectual Property & Standards

(57) **Abstract**

There is provided a portable x-ray detector (24) configured to receive x-ray radiation (20) emitted from an x-ray source (18) and to generate signals representing properties of the x-ray radiation impacting a plurality of pixels (202) of a pixel array (200) of the detector. The detector comprises user interface circuitry (300; 400; 500) configured to obtain at least one quality indicator indicative of a quality aspect of an image produced using the generated signals and to output the quality indicator to the user.

## Description

### FIELD OF THE INVENTION

The invention relates to a portable x-ray detector and to a method of using the same. In particular, the invention relates to image quality feedback on a portable detector.

### BACKGROUND OF THE INVENTION

Automatically assessing the quality of an acquired x-ray image by analysis of the image and providing this information to the operator immediately is believed to improve the overall efficiency in radiography. Displaying the information on the screen of the imaging console or by way of a separate tablet may, however, be sub-optimal.

### SUMMARY OF THE INVENTION

There is therefore a need for more effective ways of providing immediate feedback on image quality for rationalizing medical imaging. This need is met by the subject-matter of the independent claims. Optional features are set forth by the dependent claims.

According to a first aspect, there is provided a portable x-ray detector configured to receive x-ray radiation emitted from an x-ray source and to generate signals representing properties of the x-ray radiation impacting a plurality of pixels of a pixel array of the detector. The detector comprises user interface circuitry configured to obtain at least one quality indicator indicative of a quality aspect of an image produced using the generated signals and to output the quality indicator to the user.

According to a second aspect, there is provided an imaging system comprising the portable x-ray detector of the first aspect and the x-ray source. The imaging system may further comprise the image processing circuitry described herein.

According to a third aspect, there is provided a method of using a portable x-ray detector configured to receive x-ray radiation emitted from an x-ray source and to generate signals representing properties of the x-ray radiation impacting a plurality of pixels of a pixel array of the detector. The method comprises operating user interface circuitry of the detector to obtain at least one quality indicator indicative of a quality aspect of an image produced using the generated signals and to output the quality indicator to the user.

The properties of the x-ray radiation represented by the signals may include one or more of intensity, phase (phase contrast imaging), and angle of impact (darkfield imaging).

These and other aspects of the invention will be apparent from and elucidated with reference to the embodiments described hereinafter.

### BRIEF DESCRIPTION OF THE DRAWINGS

A detailed description will now be given, by way of example only, with reference to the accompanying drawings, in which:
Fig. 1 schematically illustrates an example of a medical x-ray imaging system including an x-ray detector;
Fig. 2 schematically illustrates a pixel array of the detector of Fig. 1;
Fig. 3 illustrates the detector of Fig. 1 comprising user interface circuitry outputting a binary quality indicator;
Fig. 4 illustrates the detector of Fig. 1 comprising user interface circuitry outputting a quality indicator indicating whether a region of interest exceeds detector limits;
Fig. 5 illustrates the detector of Fig. 1 comprising a display displaying an image produced using the imaging system;
Fig. 6 illustrates the detector of Fig. 1 comprising a display displaying a simplified version of an image produced using the imaging system;
Fig. 7 illustrates a remote display displaying an image of the detector of Fig. 1; and
Fig. 8 illustrates a computing device that can be used in accordance with the systems and methods disclosed herein.

### DETAILED DESCRIPTION OF EMBODIMENTS

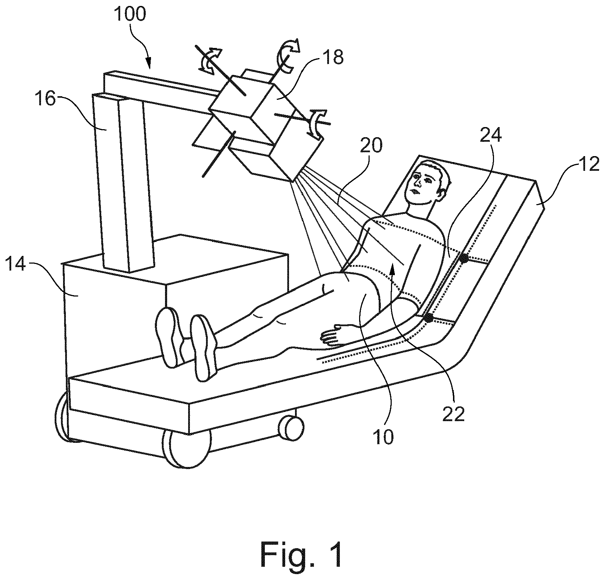

Fig. 1 illustrates a medical x-ray imaging system 100 shown in a bedside medical imaging situation. In the shown example, medical image information is acquired from a patient 10 positioned on a patient support 12. The imaging system 100 comprises a movable base unit 14, a support arm 16, and an x-ray source 18 shown here in the form of a spatially adjustable x-ray tube. The source 18 generates and emits an x-ray beam 20 towards the patient 10 and irradiates a region of interest 22. An x-ray detector 24 is positioned between the patient support 12 and the region of interest 22. The detector 24 is configured to receive the x-ray radiation 20 emitted from the source 18 and to generate signals representing the intensity of the x-ray radiation impacting a plurality of pixels 202 of a pixel array 200 of the detector, as described further below. The detector 24 can be, for example, a portable flat panel detector, which can have a cable or wireless data connection and/or a power supply connection with the base unit 14. The source 18 may be configured to deliver the radiation 20 at a plurality of energy levels, and the detector 24 may be configured to generate image data in response to radiation 20 at different energy levels. The source 18 may include a collimator (not shown) for adjusting a shape of the x-ray beam 20.

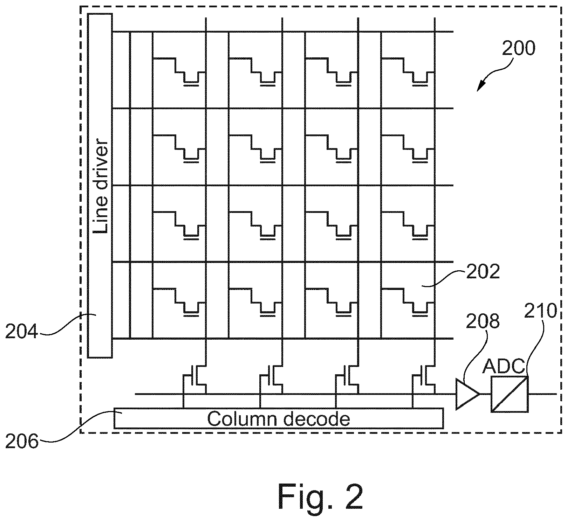

Fig. 2 schematically illustrates the pixel array 200 of the detector 24. The detector 24 comprises a scintillator (not shown) that converts x-ray photons received on the detector surface during medical imaging to lower energy (light) photons. The pixel array 200 takes the form of an amorphous silicon active matrix comprising a two-dimensional array of pixels 202 arranged in lines (rows) and columns which convert the light photons to electrical signals, which are representative of the number of photons or the intensity of radiation impacting individual pixels 202 of the array 200. Each pixel 202 comprises a photodiode and a transistor. All pixels 202 of one line are connected to the same address line that is set to a certain voltage by a line driver 204 when the pixels 202 of that line are to be addressed. The pixels 202 of each column are connected to the same readout line, which leads to a column decoder 206, which is in turn connected to a charge amplifier 208 and to an analog-to-digital converter 210 to amplify and digitize the signals on that readout line. During a readout, the line driver 204 consecutively addresses the lines of pixels 202 in the array, and in each step the signals of each line are passed to the column decoder 206 via the readout lines, such that the signals are read out line by line from the first line to the last line. The signals so read out are then passed to image processing circuitry configured to generate an image for display. In this example, the image processing circuitry forms part of an imaging console (not shown), which in turn forms part of the base unit 14. In this example, the image processing circuitry is further configured to perform image analysis for quality aspects. Any suitable quality analysis or quality assessment may be performed including those according to international standards. For example, the image analysis may be performed in the manner described in "Robust chest x-ray quality assessment using convolutional neural networks and atlas regularization", by Jens von Berg et al, Proceedings Volume 11313, Medical Imaging 2020: Image Processing; 113131L (2020).

In preparation for medical imaging, the detector 24 is manually positioned behind the region of interest 22. In order to achieve a satisfying image quality, the source 18 and the detector 24 need to be correctly aligned in terms of angular orientation, centring, and the correct source-detector distance. Therefore, it may become necessary for the radiologist to adjust a position of the source 18 such that it comes into a defined arrangement with the detector 24. This can be achieved, for instance, by manual shifting and/or turning the source 18, the detector 24, or both detector 24 and source 18. Instead of moving the detector 24 separately, also the entire arrangement of the patient support 12, the detector 24, and the patient 10 can be moved. To minimize the amount of time needed for proper positioning of the detector 24 relative to the source 18, it is beneficial to provide immediate feedback on the image quality which is attainable at the current detector position.

Proposed herein are devices and methods for indicating quality aspects directly at the detector 24, which is handled anyway during image acquisition and to which the radiologist pays most attention. Aspects of the image quality are directly displayed on the detector 24 or at its edge using one or more quality indicators. This happens after the image acquisition and after the image has been analysed for quality aspects. To this end, the detector 24 comprises user interface circuitry configured to obtain at least one quality indicator indicative of a quality aspect of the image produced as described above and to output the quality indicator to the user.

Various quality aspects or quality criteria are considered by the present disclosure. For example, as described further below, the quality indicator may relate to positioning of the detector (24) relative to the source (18). Additionally or alternatively, the quality indicator may indicate whether collimation in the image satisfies one or more predetermined acceptability criteria. Other examples will be apparent to the person skilled in the art. Moreover, numerous forms of quality indicator are envisaged by the present disclosure including for example numbers, binary (ok, not ok), ternary (traffic light) or analogue (e.g. slider) representations. Various non-limiting examples of quality indicators will now be described in more detail.

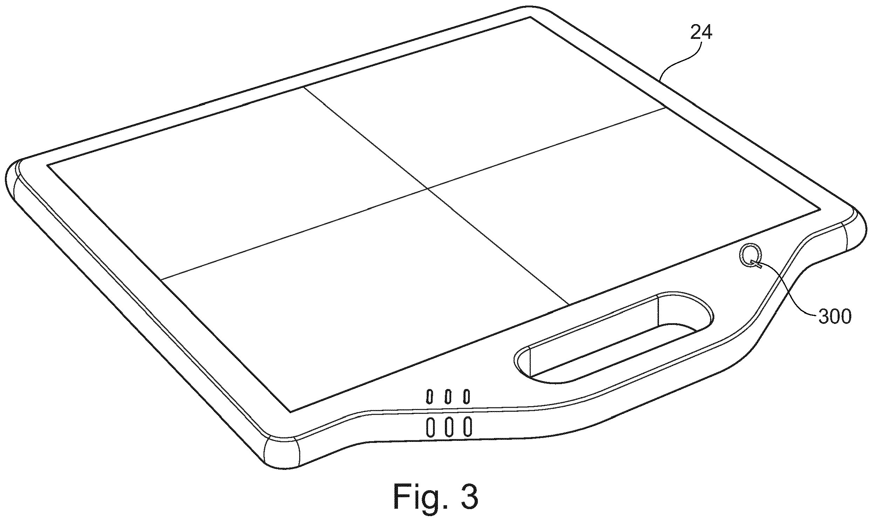

Fig. 3 illustrates the detector 24 comprising user interface circuitry 300 outputting a binary quality indicator, i.e. a quality indicator which provides a binary indication of image quality. In this example, the user interface circuitry 300 comprises an LED in the shape of the letter "Q", which is illuminated e.g. green to indicate acceptable image quality. The letter "Q" may remain unilluminated or may be illuminated with e.g. a red colour to indicate unacceptable image quality. It will be appreciated that other shapes and colours are possible. In another embodiment, the quality indicator provides a ternary indication of image quality using e.g. a traffic light system. In yet another embodiment, more detailed information is provided by LEDs such as "image okay" versus "image not okay" lighting up e.g. one second after the acquisition has been performed.

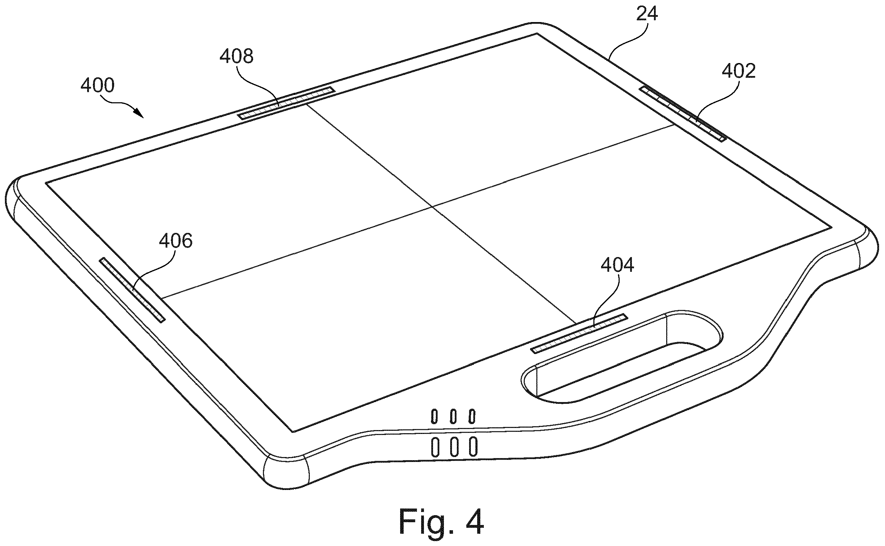

Fig. 4 illustrates the detector 24 comprising user interface circuitry 400 outputting a quality indicator indicating whether a region of interest in the image exceeds one or more detector limits. The quality indicator furthermore indicates on which side or sides of the detector 24 the region of interest exceeds the detector limits. In particular, the user interface circuitry 400 comprises four LEDs 402-408 positioned at each side of the detector 24 to indicate whether the region of interest (e.g. a target organ) has exceeded a detector limit at the respective side of the image and/or whether collimation was performed well. The detector 24 is configured to activate or deactivate the LEDs 402-408 to indicate on which side or sides of the detector the region of interest exceeds the detector limits. In this example, each of the LEDs 402-408 provides a binary indication as to whether or not the respective detector limit was exceeded. For example, the LEDs may illuminate green or red to indicate that the respective detector limit has not been exceeded or has been exceeded, respectively. Again, it will be appreciated that other forms of indication such as ternary indicators are possible indicating for instance one of "okay", "suboptimal", "to be redone". By "detector limit" is meant any restriction imposed on the amount of information which may be captured. In particular, the detector limits may comprise one or more boundaries defined by the pixel array 200 of the detector 24 and/or one or more collimator edges defined by the x-ray source 18.

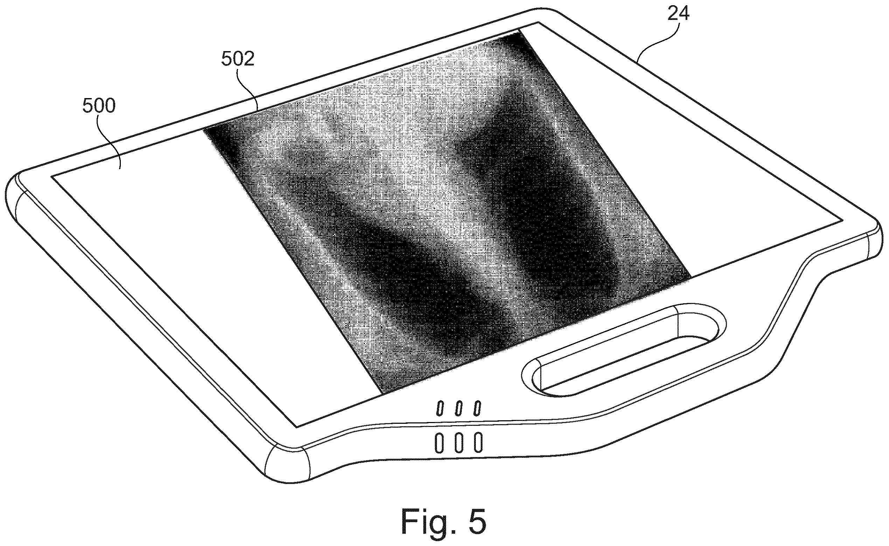

Fig. 5 illustrates the detector 24 comprising user interface circuitry in the form of a display 500 displaying an image 502 produced using the imaging system 100. Thus, in this example, the quality indicator comprises the image itself 502 displayed on the display 500. The display 500 may comprise an e-paper display. Preferably, the display 500 overlays the pixel array 200 of the detector 24, with edges of the display 500 being registered with those of the pixel array 200 so that the boundaries of the pixel array 200 can readily be indicated. However, it will be understood that other arrangements may be employed for indicating the spatial relationship between the region of interest and the array boundary (or e.g. collimator edges).

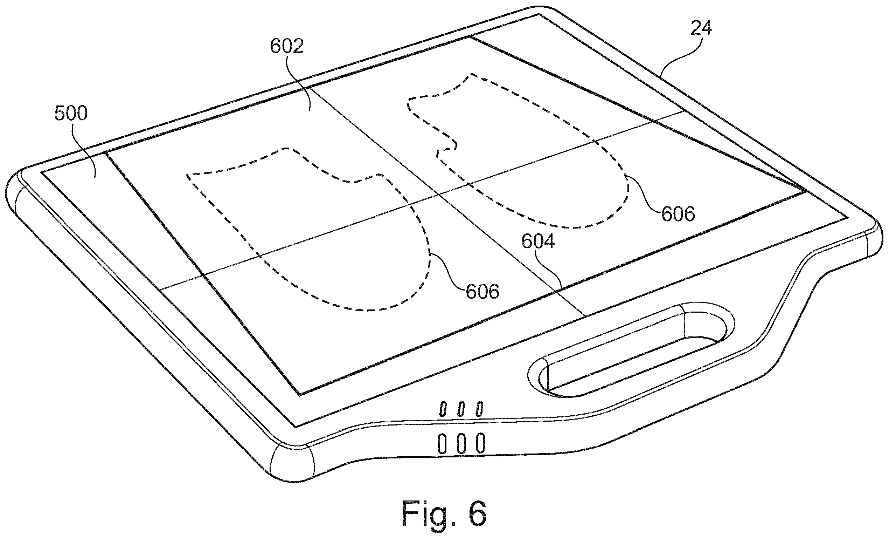

Fig. 6 illustrates the detector 24 comprising the display 500 but displaying in this example a simplified version 602 of the image 502 produced using the imaging system 100. Thus, in this example, the quality indicator comprises the simplified version 602 of the image 502 displayed on the display 500. In this way, important information derived from the image 502 may be displayed on the detector 24 to allow for assessment of patient positioning. In particular, collimator edges 604 and region of interest (e.g. target organ) contours 606 may be displayed. The contours 606 may be derived from the image 502 using for example image segmentation. Identification of regions of interest and their contours may be performed in the manner described in "Robust chest x-ray quality assessment using convolutional neural networks and atlas regularization", by Jens von Berg et al, Proceedings Volume 11313, Medical Imaging 2020: Image Processing; 113131L (2020). The detector 24 is thus configured to display on the display 500 a spatial relationship between two or more of: the collimator edges 604; the region of interest 22 or its contours 606; and the edges of the pixel array 200, to indicate among other things whether collimation was performed well and whether the detector 24 was positioned well, including whether the region of interest 22 exceeds one or more detector limits, such as the collimator edges 604 or the array boundary.

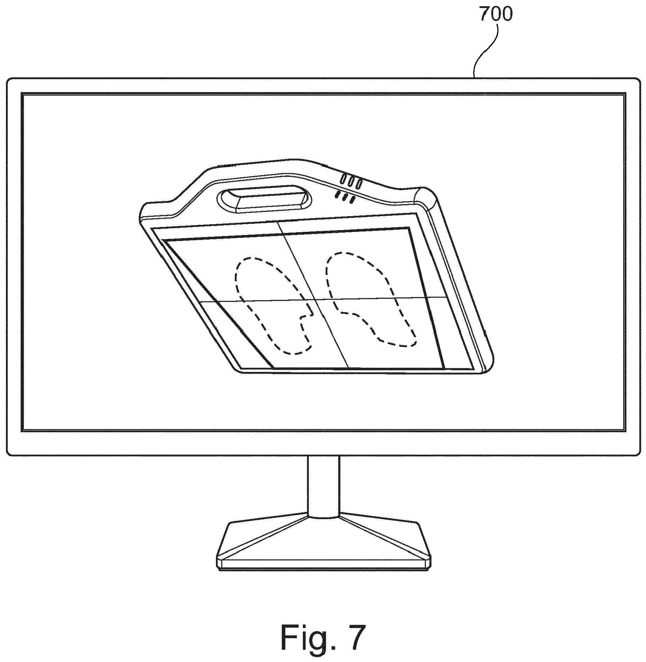

Fig. 7 illustrates a remote display 700 displaying an image of the detector 24. As the detector 24 might be at least partially covered by the patient 10, an image of the detector 24 (e.g. with the above-described anatomic outlines drawn on the detector 24) can optionally be displayed on the remote display 700, e.g. a separate monitor, such as that of the imaging console or a tablet or smartphone. This will clearly indicate how the detector position might need to be adjusted in case of a repeat image, enabling the radiologist to observe the feedback directly while managing the patient 10 in the exam room, even when the detector 24 is placed such that observing the user interface circuitry of the detector 24 is impeded by patient position.

Further provided by the present disclosure is a method of using the portable x-ray detector 24. The method comprises operating user interface circuitry of the detector 24 to obtain at least one quality indicator indicative of a quality aspect of an image 502 produced using signals generated by the detector and to output the quality indicator to the user.

The above examples relate to visual indicators of image quality. It will be understood that the user interface circuitry may additionally or alternatively provide audible and/or haptic quality indicators.

In the above examples, the image processing circuitry forms part of the imaging console. It will be understood that the image processing circuitry may equally form part of the detector 24 itself. Thus, the detector 24 may further comprise image processing circuitry configured to analyse the image to determine the at least one quality indicator indicative of a quality aspect of the generated image. The image processing circuitry may be further configured to produce the image using the generated signals. The image processing circuitry may in other examples comprise distributed circuitry. Image processing may equally be performed on a dedicated server or in the cloud.

Although, in the above example, the same image processing circuitry performs both image generation and image analysis, it will be understood that separate circuitry may be provided for these purposes.

In the above examples, the image is analysed post-acquisition for quality aspects. In other examples, image quality may be predicted before the acquisition has been performed. To this end, cameras e.g. 3D cameras or other sensors may be employed that are able to localize the patient 10, x-ray source, and the detector 24. Given these estimates of the imaging scene, properties of the resulting x-ray image can be simulated and results from this simulation can be displayed on the detector even before x-ray exposure to help operating the equipment and positioning the patient.

In the above examples, signals are read out from the main pixel array 200 of the detector 24 and used to generate the image. In other examples, a computational unit is optionally incorporated on board the detector 24 to enable rapid evaluation of the data such that no read-out from the main pixel array 200 of the detector 24 is required to generate the feedback. For instance, a dedicated minor set of detector units may be used alongside the full detector array 200 that is sufficient approximately to localize a target organ, and that can be read out more quickly than the full detector array 200. In this case, the image on which the quality assessment is performed may comprise that derived from output of the dedicated set of detector units.

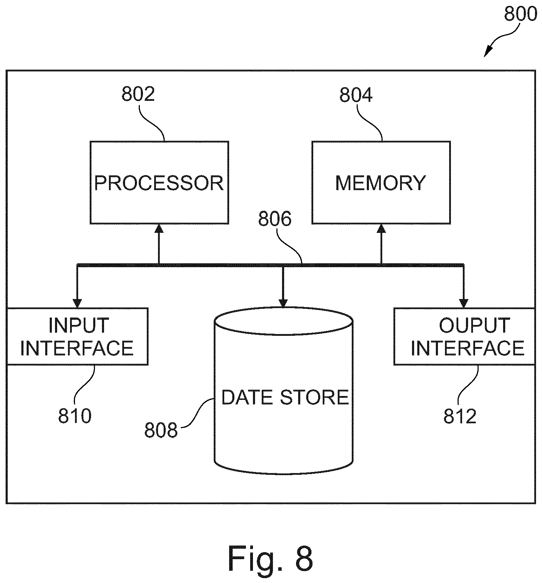

Referring now to Fig. 8, a high-level illustration of an exemplary computing device 800 that can be used in accordance with the systems and methodologies disclosed herein is illustrated. In particular, the computing device 800 may be used to implement components of the imaging console and/or the detector as described herein, including its image processing circuitry. The computing device 800 includes at least one processor 802 that executes instructions that are stored in a memory 804. The instructions may be, for instance, instructions for implementing functionality described as being carried out by one or more components discussed above or instructions for implementing one or more of the methods described above. The processor 802 may access the memory 804 by way of a system bus 806. In addition to storing executable instructions, the memory 804 may also store conversational inputs, scores assigned to the conversational inputs, etc.

The computing device 800 additionally includes a data storage 808 that is accessible by the processor 802 by way of the system bus 806. The data storage 808 may include executable instructions, log data, etc.

The computing device 800 also includes an input interface 810 that allows external devices to communicate with the computing device 800. For instance, the input interface 810 may be used to receive instructions from an external computer device, from a user, etc. The computing device 800 also includes an output interface 812 that interfaces the computing device 800 with one or more external devices. For example, the computing device 800 may display text, images, etc. by way of the output interface 812. The input and output interfaces 810, 812 may thus serve as the user interface circuitry 300, 400, 500. It is contemplated that the external devices that communicate with the computing device 800 via the input interface 810 and the output interface 812 can be included in an environment that provides substantially any type of user interface with which a user can interact. Examples of user interface types include graphical user interfaces, natural user interfaces, and so forth. For instance, a graphical user interface may accept input from a user employing input device(s) such as a keyboard, mouse, remote control, or the like and provide output on an output device such as a display. Further, a natural user interface may enable a user to interact with the computing device 800 in a manner free from constraints imposed by input device such as keyboards, mice, remote controls, and the like. Rather, a natural user interface can rely on speech recognition, touch and stylus recognition, gesture recognition both on screen and adjacent to the screen, air gestures, head and eye tracking, voice and speech, vision, touch, gestures, machine intelligence, and so forth.

Additionally, while illustrated as a single system, it is to be understood that the computing device 800 may be a distributed system. Thus, for instance, several devices may be in communication by way of a network connection and may collectively perform tasks described as being performed by the computing device 800.

Various functions described herein can be implemented in hardware, software, or any combination thereof. If implemented in software, the functions can be stored on or transmitted over as one or more instructions or code on a computer-readable medium. Computer-readable media includes computer-readable storage media. A computer-readable storage media can be any available storage media that can be accessed by a computer. By way of example, and not limitation, such computer-readable storage media can comprise RAM, ROM, EEPROM, CD-ROM or other optical disk storage, magnetic disk storage or other magnetic storage devices, or any other medium that can be used to carry or store desired program code in the form of instructions or data structures and that can be accessed by a computer. Disk and disc, as used herein, include compact disc (CD), laser disc, optical disc, digital versatile disc (DVD), floppy disk, and Blu-ray disc (BD), where disks usually reproduce data magnetically and discs usually reproduce data optically with lasers. Further, a propagated signal is not included within the scope of computer-readable storage media. Computer-readable media also includes communication media including any medium that facilitates transfer of a computer program from one place to another. A connection, for instance, can be a communication medium. For example, if the software is transmitted from a website, server, or other remote source using a coaxial cable, fibre optic cable, twisted pair, digital subscriber line (DSL), or wireless technologies such as infrared, radio, and microwave, then the coaxial cable, fibre optic cable, twisted pair, DSL, or wireless technologies such as infrared, radio and microwave are included in the definition of communication medium. Combinations of the above should also be included within the scope of computer-readable media.

Alternatively, or in addition, the functionally described herein can be performed, at least in part, by one or more hardware logic components. For example, and without limitation, illustrative types of hardware logic components that can be used include Field-programmable Gate Arrays (FPGAs), Program-specific Integrated Circuits (ASICs), Program-specific Standard Products (ASSPs), System-on-a-chip systems (SOCs), Complex Programmable Logic Devices (CPLDs), etc.

It will be appreciated that the aforementioned circuitry may have other functions in addition to the mentioned functions, and that these functions may be performed by the same circuitry.

The applicant hereby discloses in isolation each individual feature described herein and any combination of two or more such features, to the extent that such features or combinations are capable of being carried out based on the present specification as a whole in the light of the common general knowledge of a person skilled in the art, irrespective of whether such features or combinations of features solve any problems disclosed herein, and without limitation to the scope of the claims. The applicant indicates that aspects of the present invention may consist of any such individual feature or combination of features.

While the invention has been illustrated and described in detail in the drawings and foregoing description, such illustration and description are to be considered exemplary and not restrictive. The invention is not limited to the disclosed embodiments.

Other variations to the disclosed embodiments can be understood and effected by those skilled in the art, from a study of the drawings, the disclosure, and the appended claims. In the claims, the word "comprising" does not exclude other elements or steps, and the indefinite article "a" or "an" does not exclude a plurality. A single processor or other unit may fulfil the functions of several items recited in the claims. The mere fact that certain measures are recited in mutually different dependent claims does not indicate that a combination of these measures cannot be used advantageously. A computer program may be stored/distributed on a suitable medium, such as an optical storage medium or a solid-state medium supplied together with or as part of other hardware, but may also be distributed in other forms, such as via the internet or other wired or wireless communications systems. Any reference signs in the claims should not be construed as limiting the scope.

## Claims

1. A portable x-ray detector (24) configured to receive x-ray radiation (20) emitted from an x-ray source (18) and to generate signals representing properties of the x-ray radiation impacting a plurality of pixels (202) of a pixel array (200) of the detector, the detector comprising user interface circuitry (300; 400; 500) configured to obtain at least one quality indicator indicative of a quality aspect of an image (502) produced using the generated signals and to output the quality indicator to the user.

2. The detector of claim 1, wherein the quality indicator relates to positioning of the detector (24) relative to the source (18).

3. The detector of claim 1 or 2, wherein the quality indicator indicates whether a region of interest (22) in the image exceeds one or more detector limits.

4. The detector of claim 3, wherein the quality indicator further indicates on which side or sides of the detector (24) the region of interest (22) exceeds the detector limits.

5. The detector of claim 4, wherein the user interface circuitry (400) comprises output elements (402-408) positioned at each side of the detector (24), wherein the detector is configured to activate or deactivate the output elements to indicate on which side or sides of the detector the region of interest (22) exceeds the detector limits.

6. The detector of any or claims 3-5, wherein the detector limits comprise one or more boundaries defined by the pixel array (200) of the detector (24) and/or one or more collimator edges defined by the x-ray source (18).

7. The detector of any preceding claim, wherein the quality indicator indicates whether collimation in the image satisfies one or more predetermined acceptability criteria.

8. The detector of any preceding claim, wherein the quality indicator provides a binary indication of image quality or a ternary indication of image quality.

9. The detector of any preceding claim, wherein the user interface circuitry comprises a display (500), wherein the display overlays the pixel array (200) of the detector (24), and wherein edges of the display are registered with those of the pixel array.

10. The detector of claim 9, wherein the detector (24) is configured to display on the display (500) a spatial relationship between one or more collimator edges (604) and the edges of the pixel array (200).

11. The detector of any preceding claim, wherein the user interface circuitry comprises a display (500), wherein the quality indicator comprises the image itself (502) displayed on the display.

12. The detector of any of claims 1-10, wherein the user interface circuitry comprises a display (500), wherein the quality indicator comprises a simplified version (602) of the image displayed on the display.

13. The detector of any preceding claim, wherein the user interface circuitry comprises a display (500), wherein the detector (24) is configured to display on the display a region of interest (22) in the image, or a contour (606) of the region of interest.

14. An imaging system (100) comprising the portable x-ray detector (24) of any preceding claim and the x-ray source (18).

15. A method of using a portable x-ray detector (24) configured to receive x-ray radiation (20) emitted from an x-ray source (18) and to generate signals representing properties of the x-ray radiation impacting a plurality of pixels (202) of a pixel array (200) of the detector, the method comprising operating user interface circuitry (300; 400; 500) of the detector to obtain at least one quality indicator indicative of a quality aspect of an image (502) produced using the generated signals and to output the quality indicator to the user.
